# EUROPÄISCHE PATENTSCHRIFT

(11) **EP 0 811 630 B1**
(45) Veröffentlichungstag und Bekanntmachung des Hinweises auf die Patenterteilung: **14.12.2005**
(21) Anmeldenummer: 97108498.3
(22) Anmeldetag: 27.05.1997
(51) Int. Cl.: C07F 17/00, C07B 31/00, B01J 31/00, C07F 7/28

(54) **Chirale Verbindungen**
Chiral compounds
Composés chiraux

(30) Priorität: 03.06.1996 DE 19622271
(43) Veröffentlichungstag der Anmeldung: 10.12.1997
(73) Patentinhaber: Basell Polyolefine GmbH, 50389 Wesseling (DE)
(72) Erfinder: Stürmer, Rainer, Dr., 67127 Roedersheim (DE); Okuda, Jun, Prof. Dr., 55218 Ingelheim (DE); Ritter, Kurt, Dr., Newton, MA 02160 (US)

(56) Entgegenhaltungen:
- US-A- 5 264 405
- US-A- 5 621 126
- CIRUELOS, SANTIAGO ET AL: "Synthesis and Reactivity of [(Amidosilyl)cyclopentadienyl]titanium and -zirconium Complexes. X-ray Molecular Structure of [Zr{.eta.5:.eta.1- C5H4SiMe2(.mu.-O)}Cl2{H2N(CHMe)Ph}]2" ORGANOMETALLICS (1996), 15(26), 5577-5585, XP002207154
- MCKNIGHT, A.L. ET AL: "Propylene polymerization with Cp-amido metal complexes: stereoselectivity and regioselectivity" POLYM. PREPR. (AM. CHEM. SOC., DIV. POLYM. CHEM.) (1996), 37(2), 474, XP001084769
- MCKNIGHT, ANDREW L. ET AL: "Selectivity in Propylene Polymerization with Group 4 Cp-Amido Catalysts" ORGANOMETALLICS (1997), 16(13), 2879-2885, XP002207155
- OKUDA, JUN ET AL: "Optically active titanium complexes containing linked amido cyclopentadienyl ligands. Their use as asymmetric hydrogenation catalysts" CHEMISCHE BERICHTE (1996), 129(12), 1429-1431, XP001014218

## Beschreibung

Die vorliegende Erfindung betrifft chirale Verbindungen der allgemeinen Formel I 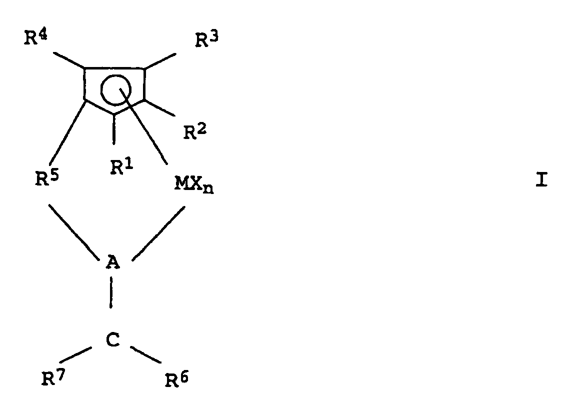 in der die Substituenten folgende Bedeutung haben:
- M: Titan, Zirkonium, Hafnium, Vanadium, Niob, Tantal oder ein Element der Lanthanidenreihe,
- X: Fluor, Chlor, Brom, Iod, Wasserstoff, C₁- bis C₁₀-Alkyl, C₆- bis C₁₅-Aryl, Alkylaryl mit 1 bis 10 C-Atomen im Alkylrest und 6 bis 20 C-Atomen im Arylrest, -OR⁸ oder -NR⁸R⁹,
wobei
- R⁸ und R⁹: C₁- bis C₁₀-Alkyl, C₆- bis C₁₅-Aryl, Alkylaryl, Arylalkyl, Fluoralkyl oder Fluoraryl mit jeweils 1 bis 10 C-Atomen im Alkylrest und 6 bis 20 C-Atomen im Arylrest bedeuten,
- R¹ bis R⁴: Wasserstoff, C₁- bis C₁₀-Alkyl, 5- bis 7-gliedrige Cycloalkyl, das seinerseits ein C₁- bis C₁₀-Alkyl als Substituent tragen kann, C₆- bis C₁₅-Aryl oder Arylalkyl, wobei gegebenenfalls auch zwei benachbarte Reste gemeinsam für 4 bis 15 C-Atome aufweisende gesättigte oder ungesättigte cyclische Gruppen stehen können oder Si(R¹⁰)₃ mit
- R¹⁰: C₁- bis C₁₀-Alkyl, C₃- bis C₁₀-Cycloalkyl oder C₆- bis C₁₅-Aryl,
- R⁵: 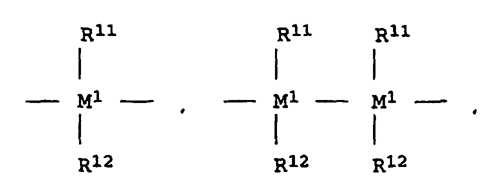 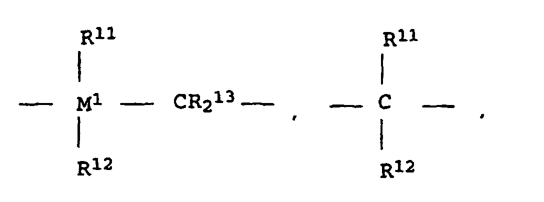 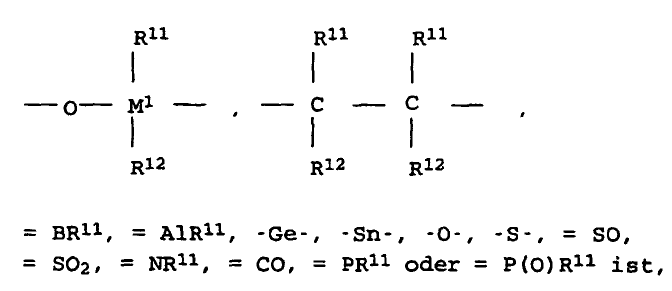
wobei
- R¹¹, R¹² und R¹³: gleich oder verschieden sind und ein Wasserstoffatom, ein Halogenatom, eine C₁-C₁₀-Alkylgruppe, eine C₁-C₁₀-Fluoralkylgruppe, eine C₆-C₁₀-Fluorarylgruppe, eine C₆-C₁₀-Arylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe oder eine C₇-C₄₀-Alkylarylgruppe bedeuten oder wobei zwei benachbarte Reste jeweils mit dem sie verbindenden Atomen einen Ring bilden, und
- M¹: Silicium, Germanium oder Zinn ist,
- A: Bor, Aluminium, Gallium, Indium, Thallium, Stickstoff, Phosphor, Arsen, Antimon oder Wismut
- R⁶ und R⁷: verschieden sind und
- R⁶: C₁- bis C₁₀-Alkyl, C₆- bis C₁₅-Aryl, Alkylaryl oder Arylalkyl mit jeweils 1 bis 10 C-Atomen im Alkylrest und 6 bis 20 C-Atomen im Arylrest, 5- bis 7-gliedriges Cycloalkyl, das seinerseits mit C₁- bis C₁₀-Alkyl oder mit Heteroatomen aufweisenden Gruppen substituiert sein kann, 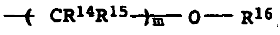 , oder 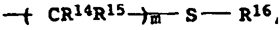 , oder -CR¹⁷R¹⁸ bedeutet, und
- R⁷: C₁- bis C₁₀-Alkyl, C₆- bis C₁₅-Aryl, Alkylaryl oder Arylalkyl mit jeweils 1 bis 10 C-Atomen im Alkylrest und 6 bis 20 C-Atomen im Arylrest, 5- bis 7-gliedriges Cycloalkyl, das seinerseits mit C₁- bis C₁₀-Alkyl oder mit Heteroatomen aufweisenden Gruppen substituiert sein kann, 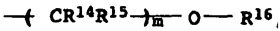 , 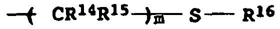 , oder -CR¹⁷R¹⁸ bedeutet, wobei R⁷ nicht Methyl oder Ethyl bedeutet,
- R¹⁴, R¹⁵ und R¹⁶: Wasserstoff, C₁- bis C₁₀-Alkyl, C₃- bis C₁₀-Cycloalkyl oder C₆- bis C₁₅-Aryl,
- R¹⁷ und R¹⁸: C₃- bis C₁₀-Cycloalkyl, C₆- bis C₁₅-Aryl oder Heteroatome aufweisende Gruppen,
- m: ganze Zahlen im Bereich von 1 bis 4
und
- n: der Wertigkeit von M abzüglich der Zahl 2 entspricht.

Weiterhin betrifft die vorliegende Erfindung Verfahren zur Herstellung solcher chiralen Verbindungen, deren Verwendung als aktive Katalysatorkomponente zur enantioselektiven Hydrierung sowie Verfahren zur enantioselektiven Hydrierung.

Für die Synthese optisch aktiver Verbindungen und chiraler Zwischenprodukte spielt die enantioselektive Hydrierung mit Rhodium- oder Rutheniumkomplexen als Katalysatoren eine große Rolle, wie in der EP-A 151 282 und der EP-A 269 395 beschrieben. Diese Rhodium- oder Rutheniumkomplexe sind jedoch verfahrenstechnisch nur aufwendig zugänglich.

In der US-A 5 292 893, US-A 5 220 020 und WO 92/09545 sind Titanverbindungen als Katalysatoren zur enantioselektiven Hydrierung beschrieben. Auch diese Verbindungen sind nur aufwendig herstellbar und müssen zudem einer Racematspaltung unterworfen werden, wobei mindestens 50 % der Substanz verlorengehen.

In der US-A 5 264 405 sind strukturell öhnlich Verbindungen und deren Verwendung zur Polymerisierung von Olefinen beschrieben.

Die bislang bekannten Verbindungen, die als Katalysatoren zur enantioselektiven Hydrierung eingesetzt werden können, machen eine industrielle Nutzung schwierig und unwirtschaftlich.

Aufgabe der vorliegenden Erfindung war es daher, neue Verbindungen zur Verfügung zu stellen, die als Katalysatoren zur enantioselektiven Hydrierung eingesetzt werden können und die die genannten Nachteile nicht aufweisen, die insbesondere verfahrenstechnisch einfach herstellbar sind.

Demgemäß wurden die eingangs beschriebenen chiralen Verbindungen der allgemeinen Formel I gefunden.

Weiterhin wurden Verfahren zur Herstellung solcher chiralen Verbindungen gefunden, deren Verwendung als aktive Katalysatorkomponente zur enantioselektiven Hydrierung sowie Verfahren zur enantioselektiven Hydrierung.

Von den chiralen Verbindungen der allgemeinen Formel I 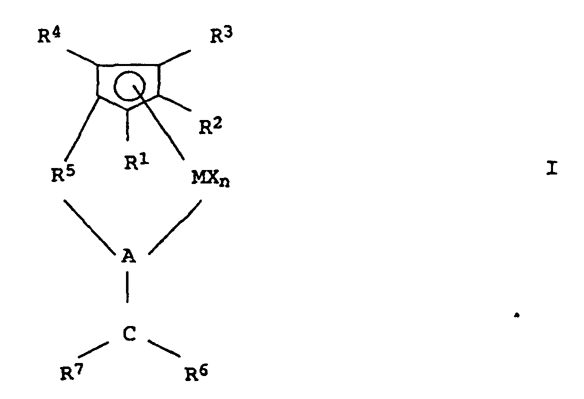 sind diejenigen bevorzugt, in denen
- M: für Titan, Zirkonium oder Hafnium steht, insbesondere für Titan,
- X: für Chlor, C₁- bis C₄-Alkyl oder Phenyl, wobei die Reste X gleich oder verschieden sein können, bevorzugt sind sie gleich,
- R¹ bis R⁴: Wasserstoff oder C₁- bis C₄-Alkyl bedeuten oder wobei zwei benachbarte Reste für 4 bis 10 C-Atome aufweisende gesättigte oder ungesättigte Gruppen stehen, so daß beispielsweise Indenyle, Tetrahydroindenyle oder Benzindenyle, die gegebenenfalls substituiert sein können, zu nennen sind,
- R⁵: für 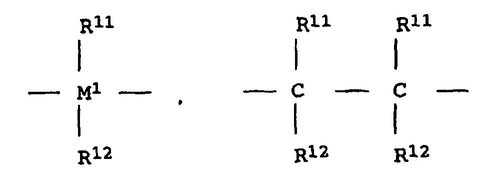 steht, insbesondere für -Si(CH₃)₂- oder -CH₂-CH₂-, aber auch für -Si(C₆H₅)₂- oder -C(CH₃)H-CH₂-,
- A: Bor, Aluminium, Stickstoff oder Phosphor bedeutet, vorzugsweise Bor, Stickstoff oder Phosphor, insbesondere Stickstoff,
- R⁶ und R⁷: die Chiralität der Verbindung ausmachen und für C₁- bis C₁₀-Alkyl, vorzugsweise C₁- bis C₄-Alkyl, insbesondere für Methyl, Ethyl, Isopropyl, sec.-Butyl oder tert.-Butyl stehen, für C₆- bis C₁₂-Aryl, insbesondere Phenyl, für Alkylaryl, insbesondere Toluolyl, für Cyclopentyl, Cyclohexyl oder mit Amingruppen substituiertes Cyclohexyl, für -CH₂OR¹⁶ oder -C(CH₃)HOR¹⁶, für -CH₂SR¹⁶ oder -CH₂-CH₂-SR¹⁶ oder oder für -C(C₆H₁₁) (NHRNR₂), wobei jedoch R⁷ nicht Methyl oder Ethyl bedeutet.
Besonders bevorzugte Gruppierungen 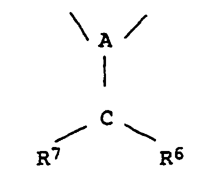 sind: 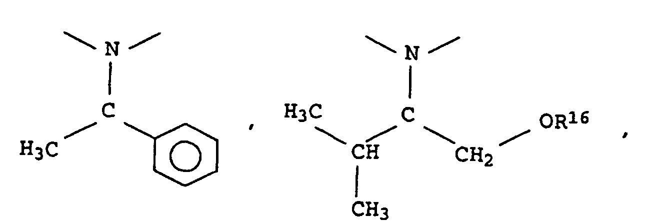 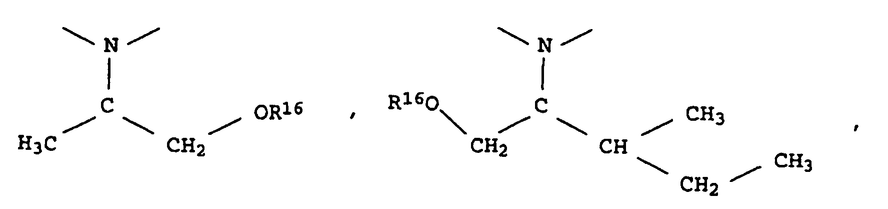 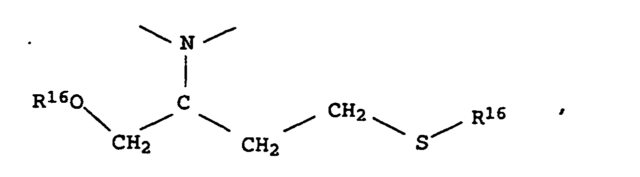 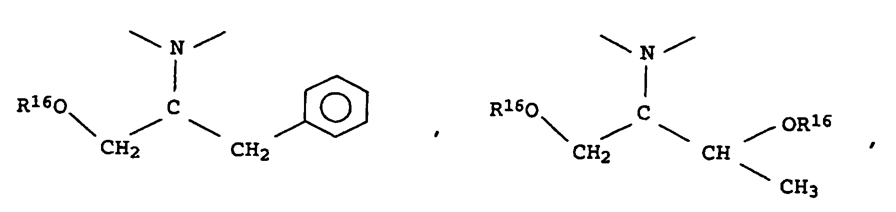 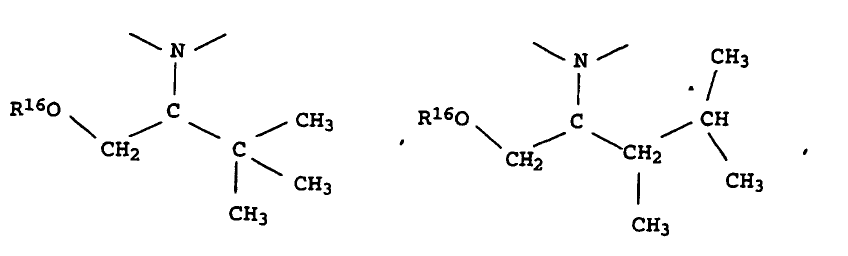 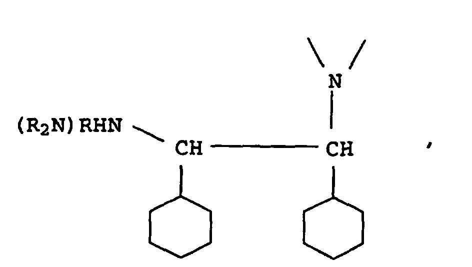 in beiden enantiomeren Formen bzw. bei Verbindungen mit weiteren Chiralitätszentren die jeweiligen Diastereomeren und Enantiomeren.

Die Reste R in diesen bevorzugten Gruppierungen stehen vorzugsweise für Methyl, Ethyl, tert.-Butyl, Benzyl, tert.-Butyl-oxycarbonyl, Benzyloxycarbonyl oder p-Methoxy-benzyl.

Die erfindungsgemäßen chiralen Verbindungen der allgemeinen Formel I können hergestellt werden, indem man die entsprechend substituierten Cyclopentadienylreste mit R⁵Z₂ und MXₙ umsetzt, wobei Z Fluor, Chlor, Brom oder Iod bedeutet und anschließend mit 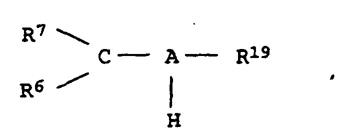 wobei

R¹⁹ Wasserstoff, ein Alkalimetall, vorzugsweise Lithium, oder M¹Y mit M¹ ein Erdalkalimetall, vorzugsweise Magnesium und Y Fluor, Chlor, Brom oder Iod bedeuten.

Die einzelnen Schritte dieser Umsetzungen sind an sich bekannt und beispielsweise in Organometallics, 14, 791 (1995) beschrieben. Die Reaktionsbedingungen sind an sich unkritisch, die Temperaturen liegen im allgemeinen im Bereich von -78 bis 50°C, bevorzugt von 0 bis 30°C. Die vorliegende erfindungsgemäße Reihenfolge der einzelnen Schritte dieser Umsetzungen weist jedoch den Vorteil der höheren Ausbeute auf.

Die erfindungsgemäßen chiralen Verbindungen der allgemeinen Formel I können als aktive Katalysatorkomponente zur enantioselektiven Hydrierung eingesetzt werden.

Als Cokatalysatoren können Aryloxyalumoxane, wie in der US-A 5,391,793 beschrieben, Aminoaluminoxane, wie in der US-A 5,371,260 beschrieben, Aminoaluminoxanhydrochloride, wie in der EP-A 633 264 beschrieben, Siloxyaluminoxane, wie in der EP-A 621 279 beschrieben, oder Mischungen daraus eingesetzt werden.

Auch Metallverbindungen wie Lithiumalkyle, Magnesiumalkyle oder Aluminiumalkyle, vorzugsweise n-Butyl-Lithium, n-Butyl-n-octyl-Magnesium, n-Butyl-n-heptyl-Magnesium, Tri-n-hexyl-aluminium, Tri-iso-butyl-aluminium, Trietylaluminium, Trimethylaluminium und Ethylmagnesiumchlorid können als Cokatalysatoren eingesetzt werden.

Bevorzugt werden n-Butyl-Lithium, Ethylmagnesiumchlorid oder Methylalumoxane eingesetzt.

Es können Mischungen verschiedener Cokatalysatoren und auch Mischungen verschiedener chiraler Verbindungen der allgemeinen Formel I eingesetzt werden.

Die Menge an chiraler Verbindungen der allgemeinen Formel I liegt vorzugsweise im Bereich von 0,001 mol-% bis 50 mol-%, bevorzugt 0,01 bis 10 mol-%, insbesondere 0,02 bis 1 mol-% bezogen auf die zu hydrierende Verbindung.

Die Menge an Cokatalysator liegt vorzugsweise im Bereich von 0,01 bis 50 Mol-Äquivalente, bevorzugt 0,5 bis 2,5 Mol-Äquivalente, insbesondere 1,5 bis 2,0 Mol-Äquivalente, bezogen auf die Menge an chiraler Verbindung der allgemeinen Formel I.

Der Katalysator kann auch in geträgerter Form eingesetzt werden, wobei Alumosilikate, Siliciumdioxide wie Kieselgele oder Kieselgure, Aktivkohle, Aluminiumoxide, Zeolithe oder Celite als Trägermaterialien zu nennen sind. Verfahren zur Trägerung von Katalysatoren sind an sich bekannt und beispielsweise in der EP-A 206 794 beschrieben.

Weiterhin können noch Additive, die die aktiven Katalysatorenkomponenten stabilisieren können, wie Silane, vorzugsweise mit mindestens einem Substituenten, der Wasserstoff ist, wie polymere Hydridosilane, polymere Zinnhydride oder Trialkylzinnhydride zugesetzt werden. Bevorzugt sind Phenylsilan, Tri-n-butyl- und Tri-n-octylzinnhydrid. Wird ein solches Additiv verwendet, so beträgt die Menge an Additiv vorzugsweise 0,1 bis 1000 Mol-Äquivalente, vorzugsweise 1 bis 10 Mol-Äquivalente, bezogen auf die Menge an chiraler Verbindung der allgemeinen Formel I.

Als Verbindungen, die hydriert werden können, sind alle diejenigen geeignet, die eine Doppelbindung besitzen, beispielsweise C=O, C=N oder C=C, mit einem oder mehreren Substituenten. Bevorzugt sind acyclische und cyclische Imine, mono-, di- und trisubstituierte Olefine, Azine, Hydrazone, Oximether, Allylether, Allylalkohole, Enamine, Enamide, Oxazolone und Ketone. Allylalkohole und Ketone werden vorzugsweise in Gegenwart von Alkyl- oder Arylsilanen als Additive hydriert. Solche Verbindungen und ihr Einsatz bei der Hydrierung sind an sich bekannt und beispielsweise in der US-A 5,292,893 und der US-A 5,491,233 beschrieben. Bevorzugt werden acyclische oder cyclische Imine eingesetzt.

Die enantioselektive Hydrierung unter Verwendung der erfindungsgemäßen chiralen Verbindungen der allgemeinen Formel I als Katalysatorkomponente wird vorzugsweise in Lösung durchgeführt. Als Lösungsmittel eignen sich alle gegenüber Organometallverbindungen stabile Lösungsmittel, beispielsweise Kohlenwasserstoffe sowie acyclische und cyclische Ether, wie Tetrahydrofuran, Toluol, Diethylether, Heptan, Hexan, Methyl-tert.butyl-ether, Dioxan, Petrolether, Dimethylether und Benzol, insbesondere Tetrahydrofuran, Toluol, Heptan oder deren Mischungen. Bei dem Einsatz von flüssigen Verbindungen, die hydriert werden, kann auch ohne Lösungsmittel gearbeitet werden.

Enantioselektive Hydrierungen sind an sich bekannt. Üblicherweise arbeitet man in einem Druckbereich von 1 bis 1000 bar, vorzugsweise 10 bis 500 bar, und in einem Temperaturbereich von -10 bis 250°C, vorzugsweise 20 bis 150°C, insbesondere 40 bis 90°C.

Zur Durchführung der enantioselektiven Hydrierung ist jeder Druckreaktor geeignet, man kann kontinuierlich oder diskontinuierlich arbeiten.

Bevorzugt geht man so vor, daß man die erfindungsgemäße chirale Verbindung der allgemeinen Formel I mit einem Lösungsmittel versetzt und bei Raumtemperatur den Cokatalysator zugibt. Anschließend wird die zu hydrierende Verbindung hinzugefügt und über einen Zeitraum von 3 bis 48 Stunden mit Wasserstoff hydriert. Danach wird der Reaktor entspannt, das Lösungsmittel entfernt und die entstandene hydrierte Verbindung gereinigt.

Wird ein Additiv verwendet, so setzt man dieses bevorzugt unmittelbar nach der Zugabe des Cokatalysators zu.

Die erfindungsgemäßen chiralen Verbindungen der allgemeinen Formel I sind verfahrenstechnisch einfach herstellbar, es ist keine Racematspaltung oder Anreicherung eines Enantiomeren erforderlich, sie eignen sich als Katalysatorkomponente bei der enantioselektiven Hydrierung, wobei bei der Hydrierung ein Enantiomerenüberschuß von bis zu 99 %, teilweise noch höher, erzielt werden kann, so daß ein äußerst wirtschaftliches und industriell anwendbares Hydrierverfahren zur Verfügung gestellt wird. Weiterhin kann eine Vielzahl von verschiedenen Verbindungen zur enantioselektiven Hydrierung eingesetzt werden.

### Beispiele

### Beispiel 1: Herstellung von {η5 ; η1 - [1 - (1'-(1R)-methylbenzylamido)-dimethylsilyl]-cyclopentadienyl}-dichlortitan (I1)

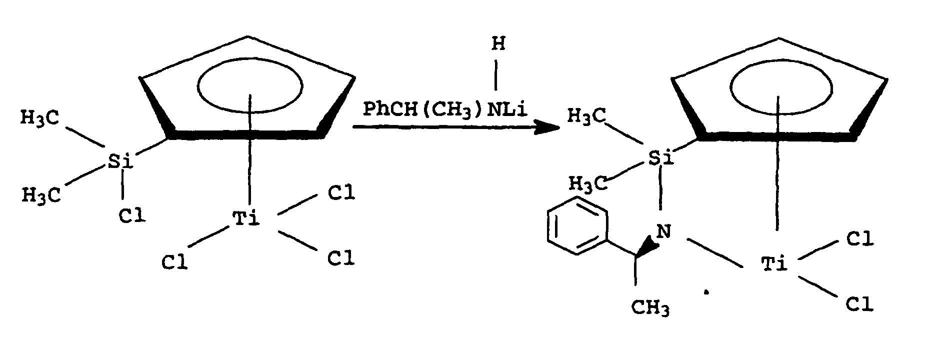

15,0 mmol Chlordimethylsilyl-cyclopentadienyl-titan(III)chlorid (Royo et. al.; Organometallics, 1995, 177) wurden in 150 ml Ether gelöst und mit 15 mmol Triethylamin versetzt. Dann wurde eine Lösung von Lithium-(1R)-1-phenylethylamid in 180 ml THF/Ether (1:1) auf -78°C gekühlt und in die ebenfalls auf -78°C gekühlte Lösung des Titankomplexes getropft. Es wurde 30 min bei dieser Temperatur und 2h bei RT gerührt. Der Ansatz wurde filtriert, die Lösung vom Lösungsmittel befreit und der Rückstand aus Ether umkristallisiert. Man erhielt den Titankomplex I1 in 72 % Ausbeute; 4,10 g gelbe Prismen.

### Spektroskopische Daten:

400 MHz H-NMR (C₆D₆)
-0.48 (s, 3H); 0.08 (s, 3H); 1.50 (d, J=6.8 Hz, 3H); 5.99-6.04 (m, 2H); 6.47-6.50 (m, 2H); 6.52 (q, J=6.8 Hz, 1H), 7.02 (m, 3H), 7.27 (m, 2H)
100 MHz C-NMR (C₆D₆):
-3.11; -0.69; 0.92; 19.12; 64.63; 109.51; 124.12; 124.44; 126.06; 126.11; 127.26; 128.06; 128.80; 144.10
MS (EI)
m/z: 106 (58 %); 241 (38 %) 344; (100 %); 346 (68 %)

| Elementaranalyse: | | | | |
|---|---|---|---|---|
| C₁₅H₁₉Cl₂NSiTi (360.2) | Ber. | C 50.00 | H 5.32 | N 3.89 |
| | gef. | C 49.87 | H 5.31 | N 3.79 |

| Drehwert: | |
|---|---|
| Komplex aus 1R-1-Phenylethylamin | +344.1° (589 nm, 22°C, c=1 in Diethylether) |
| Komplex aus 1S-1-Phenylethylamin | -344.1° (589 nm, 22°C, c=1 in Diethylether) |

### Beispiel 2

Analog wurde der enantiomere Komplex aus (1S)-1-Phenylethylamin hergestellt.

### Beispiel 3: Enantioselektive Hydrierung

In einem Schlenkrohr wurden 0,1 mmol des Komplexes I1 aus Beispiel 1 in 20 ml Toluol unter Argon gelöst und bei RT tropfenweise mit 0,2 mmol n-BuLi versetzt. Die anfangs orange gefärbte Lösung wurde braun; es wurde noch 5 min bei RT gerührt, dann wurden in einem Zug 100 mmol N-Benzylimin des Acetophenons zugegeben (Substrat/Katalysatorverhältnis 1000:1). Die so erhaltene Lösung wurde in einen Autoklaven überführt und 12 h bei 150 bar Wasserstoffdruck und 80°C gerührt. Der Autoklav wurde entspannt, das Reaktionsgemisch vom Lösungsmittel befreit und der ölige Rückstand im Kugelrohr destilliert. Es wurden 92 mmol (92 %) (1R)-N-Benzyl-1-Phenylethylamin als farbloses Öl erhalten. Nach Trifluoracetylierung wurde der Enantiomerenüberschuß gaschromatographisch zu ee=12 % bestimmt.

## Patentansprüche

1. Chirale Verbindungen der allgemeinen Formel I 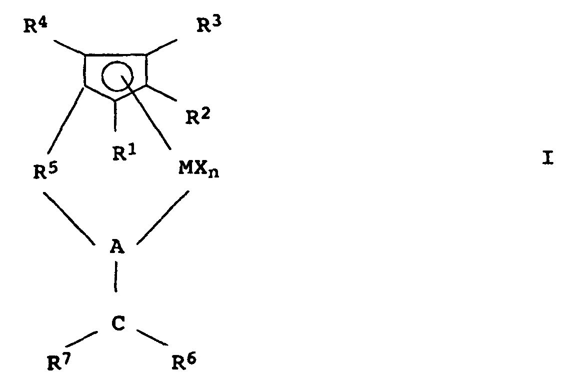 in der die Substituenten folgende Bedeutung haben:
M Titan, Zirkonium, Hafnium, Vanadium, Niob, Tantal oder ein Element der Lanthanidenreihe,
X Fluor, Chlor, Brom, Iod, Wasserstoff, C₁- bis C₁₀-Alkyl, C₆- bis C₁₅-Aryl, Alkylaryl mit 1 bis 10 C-Atomen im Alkylrest und 6 bis 20 C-Atomen im Arylrest, -OR⁸ oder -NR⁸R⁹,
wobei
R⁸ und R⁹ C₁- bis C₁₀-Alkyl, C₆- bis C₁₅-Aryl, Alkylaryl, Arylalkyl, Fluoralkyl oder Fluoraryl mit jeweils 1 bis 10 C-Atomen im Alkylrest und 6 bis 20 C-Atomen im Arylrest bedeuten,
R¹ bis R⁴ Wasserstoff, C₁- bis C₁₀-Alkyl, 5- bis 7-gliedrige Cycloalkyl, das seinerseits ein C₁- bis C₁₀-Alkyl als Substituent tragen kann, C₆- bis C₁₅-Aryl oder Arylalkyl, wobei gegebenenfalls auch zwei benachbarte Reste gemeinsam für 4 bis 15 C-Atome aufweisende gesättigte oder ungesättigte cyclische Gruppen stehen können oder Si(R¹⁰)₃ mit
R¹⁰ C₁- bis C₁₀-Alkyl, C₃- bis C₁₀-Cycloalkyl oder C₆- bis C₁₅-Aryl,
R⁵ 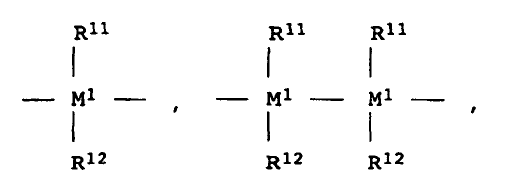 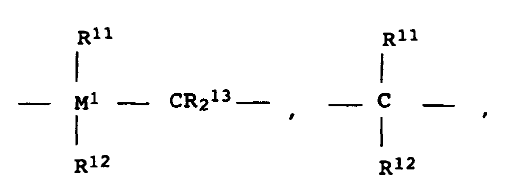 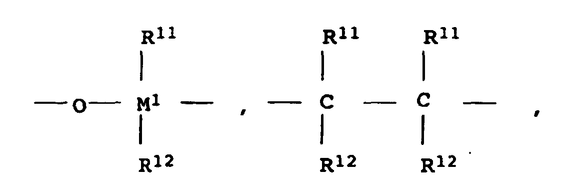 = BR¹¹, = AlR¹¹, -Ge-, -Sn-, -O-, -S-, = SO, = SO₂, = NR¹¹, = CO, = PR¹¹ oder = P(O)R¹¹ ist,
wobei
R¹¹, R¹² und R¹³ gleich oder verschieden sind und ein Wasserstoffatom, ein Halogenatom, eine C₁-C₁₀-Alkylgruppe, eine C₁-C₁₀-Fluoralkylgruppe, eine C₆-C₁₀-Fluorarylgruppe, eine C₆-C₁₀-Arylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe oder eine C₇-C₄₀-Alkylarylgruppe bedeuten oder wobei zwei benachbarte Reste jeweils mit dem sie verbindenden Atomen einen Ring bilden, und
M¹ Silicium, Germanium oder Zinn ist,
A Bor, Aluminium, Gallium, Indium, Thallium, Stickstoff, Phosphor, Arsen, Antimon oder Wismut
R⁶ und R⁷ verschieden sind und
R⁶ C₁- bis C₁₀-Alkyl, C₆- bis C₁₅-Aryl, Alkylaryl oder Arylalkyl mit jeweils 1 bis 10 C-Atomen im Alkylrest und 6 bis 20 C-Atomen im Arylrest, 5- bis 7-gliedriges Cycloalkyl, das seinerseits mit C₁- bis C₁₀-Alkyl oder mit Heteroatomen aufweisenden Gruppen substituiert sein kann, 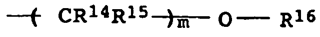 , 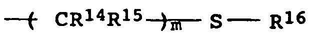 , oder -CR¹⁷R¹⁸ bedeutet, und
R⁷ C₁- bis C₁₀-Alkyl, C₆- bis C₁₅-Aryl, Alkylaryl oder Arylalkyl mit jeweils 1 bis 10 C-Atomen im Alkylrest und 6 bis 20 C-Atomen im Arylrest, 5- bis 7-gliedriges Cycloalkyl, das seinerseits mit C₁- bis C₁₀-Alkyl oder mit Heteroatomen aufweisenden Gruppen substituiert sein kann, 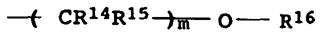 , 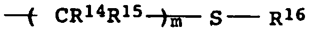 , oder ―CR¹⁷R¹⁸ bedeutet, wobei R⁷ nicht Methyl oder Ethyl bedeutet,
R¹⁴, R¹⁵ und R¹⁶ Wasserstoff, C₁- bis C₁₀-Alkyl, C₃- bis C₁₀-Cycloalkyl oder C₆- bis C₁₅-Aryl,
R¹⁷ und R¹⁸ C₃- bis C₁₀-Cycloalkyl, C₆- bis C₁₅-Aryl oder Heteroatome aufweisende Gruppen,
m ganze Zahlen im Bereich von 1 bis 4
und
n der Wertigkeit von M abzüglich der Zahl 2 entspricht.

2. Chirale Verbindungen nach Anspruch 1, in denen M in der allgemeinen Formel I für Titan, Zirkonium oder Hafnium steht.

3. Chirale Verbindungen nach den Ansprüchen 1 bis 2, in denen A in der allgemeinen Formel I für Bor, Stickstoff oder Phosphor steht.

4. Verfahren zur Herstellung von chiralen Verbindungen der allgemeinen Formel I gemäß den Ansprüchen 1 bis 3, **dadurch gekennzeichnet, daß** man die entsprechend substituierten Cyclopentadienylreste mit R⁵Z₂ und MXₙ umsetzt, wobei Z Fluor, Chlor, Brom oder Iod bedeutet und anschließend mit 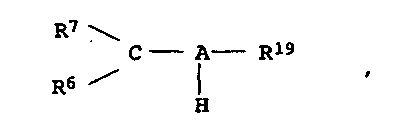 umsetzt, wobei R¹⁹ Wasserstoff, ein Alkalimetall oder M¹Y bedeutet,
mit M¹ ein Erdalkalimetall und
Y Fluor, Chlor, Brom oder Iod.

5. Verwendung von chiralen Verbindungen der allgemeinen Formel I gemäß den Ansprüchen 1 bis 3 als aktive Katalysatorkomponente zur enantioselektiven Hydrierung.

6. Verwendung von chiralen Verbindungen nach Anspruch 5, **dadurch gekennzeichnet, daß** ein Cokatalysator mitverwendet wird.

7. Verwendung von chiralen Verbindungen nach den Ansprüchen 5 bis 6 zur enantioselektiven Hydrierung von Iminen.

8. Verfahren zur enantioselektiven Hydrierung von Iminen, **dadurch gekennzeichnet, daß** man in Lösung arbeitet und als aktive Katalysatorkomponente chirale Verbindungen der allgemeinen Formel I gemäß den Ansprüchen 1 bis 3 einsetzt.

## Claims

1. A chiral compound of the formula I 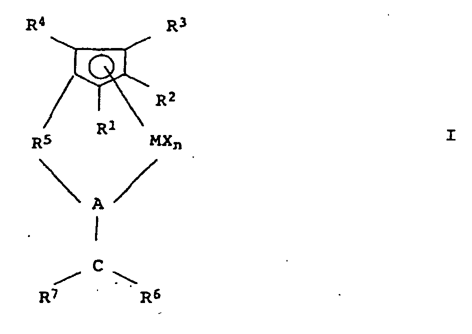 where
M is titanium, zirconium, hafnium, vanadium, niobium, tantalum or an element from the lanthanide series,
X is fluorine, chlorine, bromine, iodine, hydrogen, C₁- to C₁₀-alkyl, C₆- to C₁₅-aryl, alkylaryl having 1 to 10 carbon atoms in the alkyl radical and 6 to 20 carbon atoms in the aryl radical, -OR⁸ or -NR⁸R⁹,
where
R⁸ and R⁹ are C₁- to C₁₀-alkyl, C₆- to C₁₅-aryl, alkylaryl, arylalkyl, fluoroalkyl or fluoroaryl, in each case having 1 to 10 carbon atoms in the alkyl radical and 6 to 20 carbon atoms in the aryl radical,
R¹ to R⁴ are hydrogen, C₁- to C₁₀-alkyl, 5- to 7-membered cycloalkyl, which may itself carry a C₁- to C₁₀-alkyl as substituent, C₆- to C₁₅-aryl or arylalkyl, where, if desired, two adjacent radicals may also together be a saturated or unsaturated cyclic group having 4 to 15 carbon atoms, or Si(R¹⁰)₃, where
R¹⁰ is C₁- to C₁₀-alkyl, C₃- to C₁₀-cycloalkyl or C₆- to C₁₅-aryl, 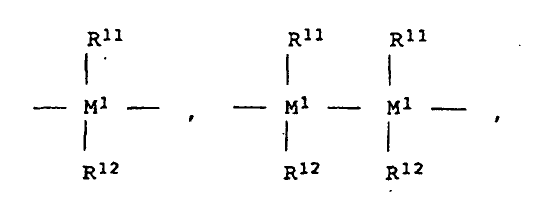 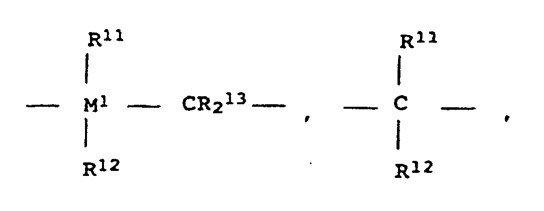 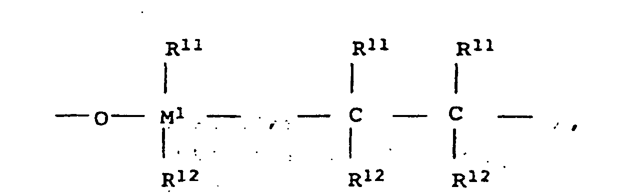
=BR¹¹, =A1R¹¹, -Ge-, -Sn-, -O-, -S-, =SO, =SO₂, =NR¹¹, =CO, =PR¹¹ or =P(O)R¹¹,
where
R¹¹, R¹² and R¹³ are identical or different and are hydrogen, halogen, C₁-C₁₀-alkyl, C₁-C₁₀- fluoroalkyl, C₆-C₁₀-fluoroaryl, C₆-C₁₀-aryl, C₁-C₁₀-alkoxy, C₂-C₁₀-alkenyl, C₇-C₄₀-arylalkyl, C₈-C₄₀-aryl-alkenyl or C₇-C₄₀-alkylaryl, or where two adjacent radicals, in each case with the atoms connecting them, form a ring, and
M¹ is silicon, germanium or tin,
A is boron, aluminum, gallium, indium, thallium, nitrogen, phosphorus, arsenic, anti-mony or bismuth,
R⁶ and R⁷ are different and
R⁶ is C₁- to C₁₀-alkyl, C₆- to C₁₅-aryl, alkylaryl or arylalkyl, in each case having 1 to 10 carbon atoms in the alkyl radical and 6 to 20 carbon atoms in the aryl radical, 5- to 7-membered cycloalkyl, which may itself be substituted by C₁- to C₁₀-alkyl or by groups containing hetero atoms, 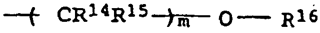 , 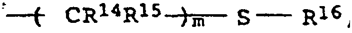 , and
R⁷ is C₁- to C₁₀-alkyl, C₆- to C₁₅-aryl, alkylaryl or arylalkyl, in each case having 1 to 10 carbon atoms in the alkyl radical and 6 to 20 carbon atoms in the aryl radical, 5- to 7-membered cycloalkyl, which may itself be substituted by C₁- to C₁₀-alkyl or by groups containing hetero atoms, 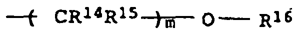 , 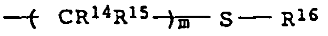 or -CR¹⁷R¹⁸ and where R⁷ is not methyl or ethyl,
R¹⁴, R¹⁵ and R¹⁶ are hydrogen, C₁- to C₁₀-alkyl, C₃- to C₁₀-cycloalkyl or C₆- to C₁₅-aryl,
R¹⁷ and R¹⁸ are C₃- to C₁₀-cycloalkyl, C₆- to C₁₅-aryl or groups containing hetero atoms,
m is an integer in the range from 1 to 4,
and
n corresponds to the valence of M minus 2.

2. The chiral compound according to claim 1 wherein M in the formula I is titanium, zirconium or hafnium.

3. The chiral compound according to claim 1 or 2 wherein A in the formula I is boron, nitrogen or phosphorus.

4. A process for the preparation of a chiral compound of the formula I according to any of claims 1 to 3, which comprises reacting an appropriately substituted cyclopentadienyl radical with R⁵Z₂ and MXₙ, where Z is fluorine, chlorine, bromine or iodine, and subsequently with 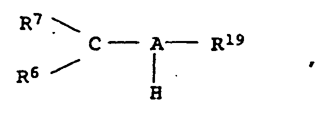 where R¹⁹ is hydrogen, an alkali metal or M¹Y,
where M¹ is an alkaline earth metal and
Y is fluorine, chlorine, bromine or iodine.

5. The use of a chiral compound of the formula I according to any of claims 1 to 3 as active catalyst component for enantioselective hydrogenation.

6. The use of a chiral compound according to claim 5 in the presence of a cocatalyst.

7. The use of a chiral compound according to claim 5 or 6 for enantioselective hydrogenation of imines.

8. A process for the enantioselective hydrogenation of imines, which comprises working in solution and using, as active catalyst component, a chiral compound of the formula I according to any of claims 1 to 3.

## Revendications

1. Composés chiraux de formule générale I 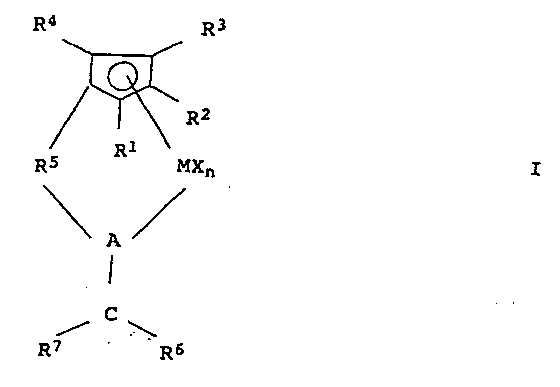 dans laquelle les symboles ont les significations suivantes :
M le titane, le zirconium, le hafnium, le vanadium, le niobium, le tantale ou un élément de la série des lanthanides,
X le fluor, le chlore, le brome, l'iode, l'hydrogène, un groupe alkyle en C₁-C₁₀, aryle en C₆ - C₁₅, alkylaryle à 1 à 10 atomes de carbone dans la partie alkyle et 6 à 20 atomes de carbone dans la partie aryle, -OR⁸ ou -NR⁸R⁹,
R⁸ et R⁹ représentant des groupes alkyle en C₁-C₁₀, aryle en C₆-C₁₅, alkylaryle, arylalkyle, fluoralkyle ou fluoraryle contenant chacun 1 à 10 atomes de carbone dans la partie alkyle et 6 à 20 atomes de carbone dans la partie aryle,
R¹ à R⁴ l'hydrogène, des groupes alkyle en C₁-C₁₀, cycloalkyle à 5 à 7 chaînons et qui peuvent porter eux-mêmes un substituant alkyle en C₁-C₁₀, aryle en C₆-C₁₅ ou arylalkyle, deux groupes voisins pouvant éventuellement former ensemble un groupe cyclique saturé ou insaturé de 4 à 15 atomes de carbone, ou Si(R¹⁰)₃ dans lequel
R¹⁰ représente un groupe alkyle en C₁-C₁₀, cycloalkyle en C₃-C₁₀ ou aryle en C₆-C₁₅,
R⁵ 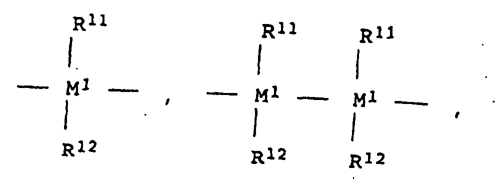 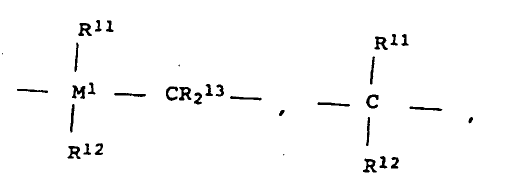 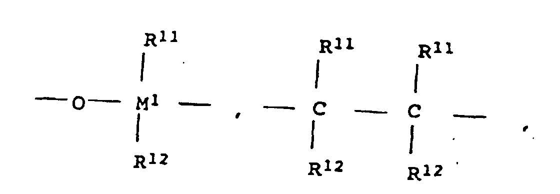 = BR¹¹, = AlR¹¹ , -Ge-, -Sn-, -O-, -S-, = SO, = SO₂, = NR¹¹, = CO, = PR¹¹ ou = P(O)R¹¹,
dans lesquels,
R¹¹, R¹² et R¹³ ayant des significations identiques ou différentes représentent chacun un atome d'hydrogène, un atome d'halogène, un groupe alkyle en C₁-C₁₀, fluoralkyle en C₁-C₁₀, fluoraryle en C₆-C₁₀, aryle en C₆-C₁₀, alcoxy en C₁-C₁₀, alcényle en C₂-C₁₀, arylalkyle en C₇-C₄₀, arylalcényle en C₈-C₄₀ ou alkylaryle en C₇-C₄₀, deux groupes voisins pouvant également former un cycle avec l'atome qui les relie, et
M¹ le silicium, le germanium ou l'étain,
A le bore, l'aluminium, le gallium, l'indium, le thallium, l'azote, le phosphore, l'arsenic, l'antimoine ou le bismuth,
R⁶ et R⁷, ayant des significations différentes, représentent respectivement :
R⁶ un groupe alkyle en C₁-C₁₀, aryle en C₆-C₁₅, alkylaryle ou arylalkyle contenant chacun 1 à 10 atomes de carbone dans la partie alkyle et 6 à 20 atomes de carbone dans la partie aryle, cycloalkyle à 5 à 7 chaînons, lequel peut être substitué par un groupe alkyle en C₁-C₁₀ ou par des groupes contenant des hétéroatomes, 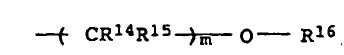 , 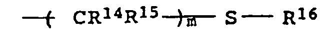 , ou -CR¹⁷R¹⁸
et
R⁷ un groupe alkyle en C₁-C₁₀, aryle en C₆-C₁₅, alkyaryle ou aryalkyle contenant chacun 1 à 10 atomes de carbone dans la partie alkyle et 6 à 20 atomes de carbone dans la partie aryle, cycloalkyle à 5 à 7 chaînons, lequel peut être substitué par un groupe alkyle en C₁-C₁₀ ou par des groupes contenant des hétéroatomes, 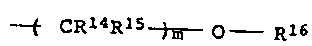 , 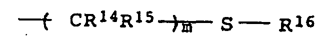 , ou -CR¹⁷R¹⁸
R⁷ ne pouvant représenter un groupe méthyle ou éthyle,
R¹⁴, R¹⁵ et R¹⁶ l'hydrogène, des groupes alkyle en C₁-C₁₀, cycloalkyle en C₃-C₁₀ ou aryle en C₆-C₁₅,
R¹⁷ et R¹⁸ des groupes cycloalkyle en C₃-C₁₀, aryle en C₆-C₁₅ ou des groupes contenant des hétéroatomes,
m des nombres entiers allant de 1 à 4
et
n la valence de M diminuée de 2.

2. Composés chiraux selon la revendication 1, pour lesquels M, dans la formule générale I, représente le titane, le zirconium ou le hafnium.

3. Composés chiraux selon les revendications 1 à 2, pour lesquels A, dans la formule générale I, représente le bore, l'azote ou le phosphore.

4. Procédé pour la préparation de composés chiraux de formule générale I selon les revendications 1 à 3, **caractérisé en ce que** l'on fait réagir les radicaux cyclopentadiényle substitués correspondants avec R⁵Z₂ et MXₙ, Z représentant le fluor, le chlore, le brome ou l'iode, puis on fait réagir avec 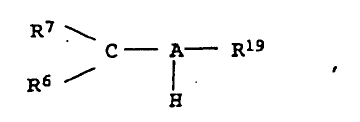
R¹⁹ représentant l'hydrogène, un métal alcalin ou M¹Y,
M¹ un métal alcalinoterreux et
Y le fluor, le chlore, le brome ou l'iode.

5. Utilisation des composés chiraux de formule générale I selon les revendications 1 à 3 en tant que composant actif de catalyseur pour d'hydrogénation énantiosélective.

6. Utilisation des composés chiraux selon la revendication 5, **caractérisée en ce que** l'on utilise conjointement un catalyseur auxiliaire.

7. Utilisation des composés chiraux selon les revendications 5 à 6 pour l'hydrogénation énantiosélective des imines.

8. Procédé pour l'hydrogénation énantiosélective des imines, **caractérisé en ce que** l'on opère en solution et on utilise des composés chiraux de formule générale I selon les revendications 1 à 3 en tant que composants actifs de catalyseurs.
